Europäisches Patentamt

European Patent Office

Office européen des brevets

(11) Numéro de publication: **0 420 715 A1**

## (12) DEMANDE DE BREVET EUROPEEN

(21) Numéro de dépôt: 90402523.6

(51) Int. Cl.⁵: **A61B 1/24**

(22) Date de dépôt: **13.09.90**

(30) Priorité: **19.09.89 FR 8912613**

(43) Date de publication de la demande:
**03.04.91 Bulletin 91/14**

(84) Etats contractants désignés:
**BE CH DE ES GB IT LI NL**

(71) Demandeur: **Mouyen, Francis**
**Chemin Gojousse, Vieille-Toulouse**
**F-31320 Castanet-Tolosav(FR)**

(72) Inventeur: **Mouyen, Francis**
**Chemin Gojousse, Vieille-Toulouse**
**F-31320 Castanet-Tolosav(FR)**

(74) Mandataire: **Morelle, Guy Georges Alain**
**CABINET MORELLE & BARDOU, SC 5,**
**Boulevard de la Méditerranée**
**F-31400 Toulouse(FR)**

(54) **Dispositif pour la prise de vues de corps masqués en totalité ou en partie, utilisable notamment dans le domaine dentaire.**

(57) L'invention concerne un dispositif destiné à la prise de vues ou à la transmission d'images de corps masqués en totalité ou en partie, ledit dispositif comprenant un miroir 1 disposé à l'extrémité 2a d'un manche 2, une source de lumière focalisée principalement vers ledit miroir et une caméra 3 montée sur ledit manche avec son axe de visée sensiblement dirigé vers ladite extrémité.

Ce dispositif est remarquable en ce que ladite extrémité 2a du manche 2 comporte une articulation 5a permettant de positionner ledit miroir 1 dans différents plans compris dans une plage angulaire allant de 200 à 90° par rapport au plan I contenant ledit manche.

Le dispositif de l'invention trouve une application dans le domaine dentaire où il peut être utilisé pour la prise et la transmission d'images de l'intérieur de la cavité buccale.

fig.1

EP 0 420 715 A1

## DISPOSITIF POUR LA PRISE DE VUES DE CORPS MASQUES EN TOTALITE OU EN PARTIE, UTILISABLE NOTAMMENT DANS LE DOMAINE DENTAIRE

La présente invention intéresse les dispositifs destinés à la prise de vues ou à la transmission d'images de corps dissimulés, en totalité ou en partie, à la vision directe de l'oeil humain ou de l'objectif d'une caméra. Ces dispositifs peuvent notamment être utilisés pour photographier ou filmer des corps situés dans des lieux difficilement accessibles et, plus particulièrement dans le domaine dentaire, pour obtenir des images de l'intérieur de la cavité buccale.

Les progrès récents dans la miniaturisation permettent en effet l'introduction de certains appareils d'enregistrement et de transmission des images directement en bouche. On connaît ainsi un dispositif d'exploration dentaire qui consiste en une caméra endoscopique montée sur un bras articulé relié à un moniteur vidéo. On connaît également un dispositif utilisant une caméra vidéo miniature qui est fixée sur le manche d'un miroir dentaire classique et à laquelle est associée une source de lumière constituée par une fibre optique elle-même montée sur le manche du miroir. Le praticien dirige ainsi l'objectif de la caméra sur la portion réflectrice du miroir sur laquelle est focalisé le faisceau lumineux fourni par la fibre optique, ce qui lui permet notamment de saisir des images des parties postérieures des dents.

Bien que d'un intérêt certain, ce dernier dispositif présente quelques inconvénients liés entre autre au fait que le miroir dentaire classique ne présente pas une structure particulièrement adaptée à un tel usage. En effet, le manque de mobilité relative du miroir et de l'objectif de la caméra limite dans des proportions importantes le champ possible de vision et donc la liberté de prise de vues et ne permet des d'obtenir des images exploitables de manière optimale de certaines parties de la cavité buccale d'accès difficile. Ceci explique qu'un tel appareil est nécessairement équipé d'une source de lumière additionnelle, le praticien ne pouvant inspecter la cavité buccale à la lumière naturelle ou à l'aide du seul scialytique utilisé couramment dans les cabinets dentaires.

On notera d'autre part qu'il en est de même avec le premier dispositif évoqué ci-dessus. Celui-ci exige en effet également l'emploi d'une source de lumière propre. Il en résulte bien évidemment une aggravation du coût de ces matériels.

Le dispositif objet de la présente invention ne présente pas de tels inconvénients. En effet, sa structure, particulièrement simple et économique quant à son prix de revient industriel, facilite et optimise l'interaction des éléments qui le composent ou qui interviennent dans son fonctionnement.

Conformément à l'invention, ce dispositif, qui comprend un miroir disposé à l'extrémité d'un manche, une source de lumière focalisée principalement vers ledit miroir et une caméra montée sur ledit manche avec son axe de visée sensiblement dirigé vers ledite extrémité, est remarquable en ce que ladite extrémité du manche comporte une première articulation permettant de positionner ledit miroir dans différents plans compris dans une plage angulaire allant de 200 à 90° par rapport au plan contenant ledit manche.

Ainsi, en donnant au miroir une mobilité suffisante on améliore grandement les facultés d'exploration de la caméra sans qu'il soit nécessaire d'utiliser une source de lumière autre que celle constituée notamment par un scialytique, bien que le dispositif de l'invention puisse être employé avec une source de lumière qui lui soit propre comme les dispositifs de l'art antérieur.

Avec une source de lumière indépendante, il suffit d'orienter le miroir de manière adéquate pour que ce dernier puisse à la fois capter le maximum de flux lumineux extérieur et fournir sans distorsion quelconque le reflet, par exemple, de la face postérieure d'une dent examinée.

En outre, en positionnant le miroir dans un plan formant un angle voisin de 200° avec le plan contenant le manche, il est possible à l'objectif de la caméra de saisir directement l'image de l'objet inspecté sans qu'il soit nécessaire de désolidariser la caméra de son support constitué par ledit manche; dans ce mcde opératoire le miroir étant en quelque sorte escamoté.

Pour ce faire, l'axe de visée de cette caméra sera de préférence situé dans un second plan parallèle au plan contenant le susdit manche pour que ledit axe n'intercepte pas l'extrémité du manche. De cette manière également, la caméra n'étant pas inclinée vers le manche, elle ne constituera pas un écran important au passage de la lumière provenant d'une source extérieure.

Les autres caractéristiques et avantages de la présente invention seront exposés dans la suite de ce mémoire qui donne un exemple de réalisation d'un dispositif conçu conformément aux enseignements de ladit invention, cet exemple étant fourni à titre simplement illustratif et ne pouvant en aucun cas être interprété de manière à limiter le champ de la protection.

La description de cet exemple se réfère à des dessins sur lesquels:

Les Figures 1 et 2 sont des vues schématiques longitudinales du dispositif en question, montrant deux positions possibles du miroir;

La Figure 3 est une vue de face de l'extrémité du manche munie de son miroir; et

La Figure 4 est une vue de détail montrant l'articulation du miroir sur le manche.

Le dispositif ainsi illustré est plus particulièrement destiné à être utilisé comme instrument dentaire.

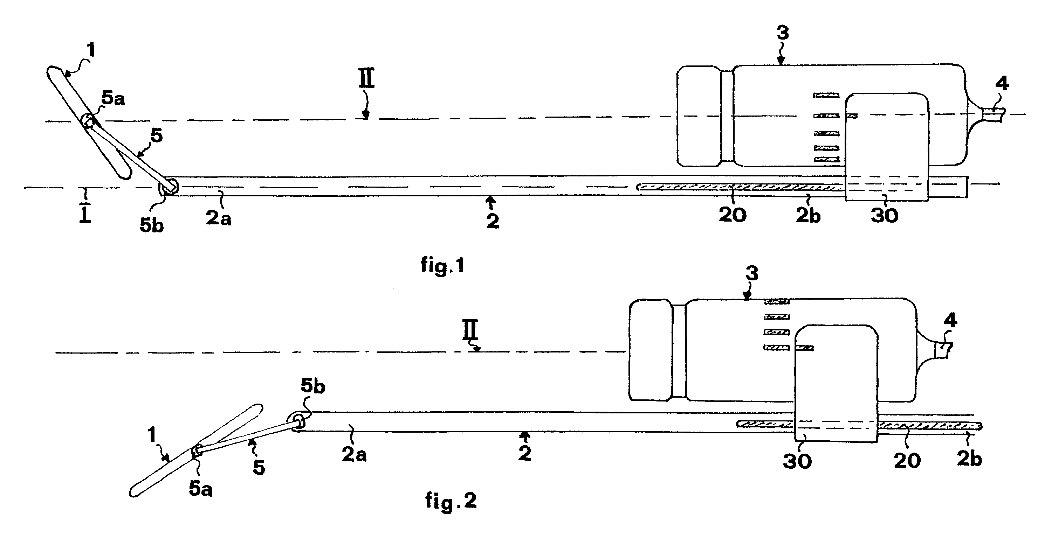

Comme on peut le voir aux Figures 1 et 2, il comprend un miroir 1 disposé à l'extrémité 2a d'un manche droit 2 et une caméra miniature 3 du type vidéo montée également sur le manche 2 au voisinage de l'extrémité 2b de ce dernier et reliée par un cable 4 à un moniteur non représenté.

Dans le mode de réalisation donné en exemple, le dispositif fonctionne avec une source de lumière indépendante qui sera constituée par un scialytique conventionnel qui n'est pas illustré mais qui sera sensiblement situé au-dessus de la caméra 3, le faisceau lumineux produit par ce scialytique étant principalement dirigé vers le miroir 1.

Conformément aux enseignements de l'invention, l'extrémité 2a du manche 2 comporte une première articulation 5a permettant de positionner le miroir 1 dans différents plans compris dans une plage angulaire allant de 200 à 90° par rapport au plan I contenant le manche 2. On compendra toutefois que le positionnement du miroir dans un plan formant un angle droit avec celui contenant le manche ne présente qu'un intérêt limité dans le domaine de l'exploration dentaire. Aussi, pour ce type d'application, la plage angulaire utile pourra être comprise entre 200 et 110° C.

Ainsi qu'on peut l'observer, l'articulation 5a autour de laquelle le miroir 1 peut pivoter, est située au centre de ce dernier et la caméra 3 est fixée sur le manche 2 de telle sorte que son axe de visée soit situé dans un second plan II, parallèle au premier plan I contenant le manche 2, et passe par le centre dudit miroir au niveau de l'articulation 5a.

De cette manière, l'objectif de la caméra pourra saisir la totalité de l'image reflétée par le miroir quelle que soit la position donnée à ce dernier dans ces plans formant un angle non nul avec le second plan II.

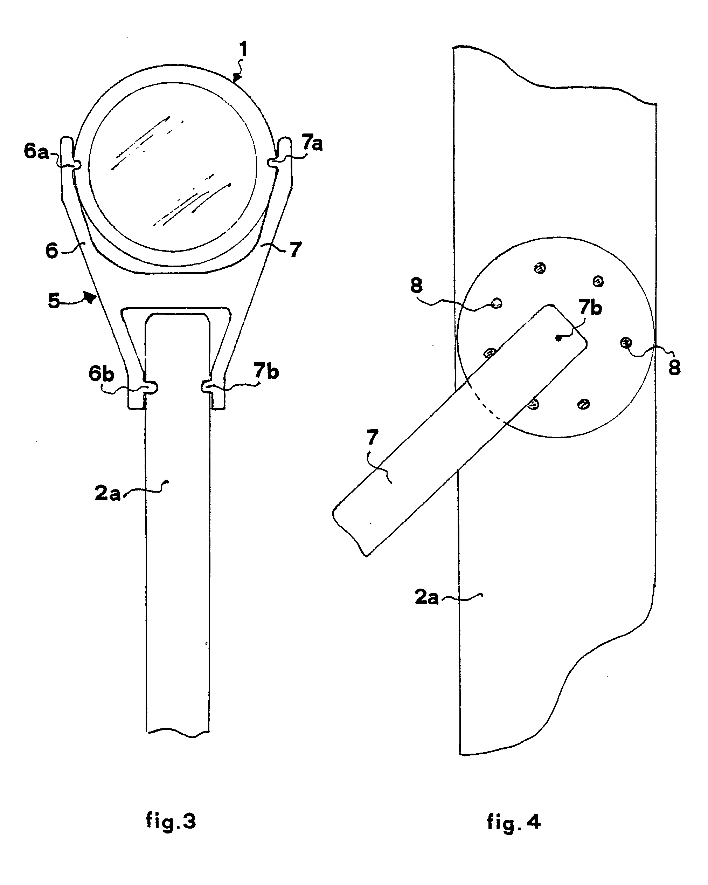

En se reportant plus particulièrement à la Figure 3, on peut voir le miroir 1, qui possède une forme circulaire, et l'extrémité 2a du manche sur laquelle est monté un étrier 5 dont les deux branches 6 et 7 sont fixées au niveau de l'axe médian du miroir au moyen de tétons 6a, 7a logés dans des orifices correspondants ouverts dans l'épaisseur du cadre dudit miroir. Cet assemblage constitue l'articulation 5a évoquée ci-dessus, le miroir pouvant ainsi être mû autour de l'axe de rotation formé par l'association des deux tétons 6a et 7a.

Dans le mode de réalisation donné à titre d'exemple, l'extrémité 2a du manche 2 comporte une seconde articulation 5b au niveau de la fixation des branches de l'étrier 5 au manche lui-même.

Cette seconde articulation est constituée par des tétons 6b, 7b portés respectivement par les branches 6 et 7 de l'étrier et qui sont logés dans des orifices correspondants ouverts sur les faces latérales du manche 2.

Cette articulation 5b permet, comme le montre la Figure 2, d'amener le miroir 1 hors du plan II contenant l'axe de visée de la caméra afin de réaliser des vues directes des dents mais aussi afin d'écarter lors des séances de prise de vues, les parties molles de la cavité buccale, par exemple la langue, avec la partie postérieure dudit miroir.

A cet effet, et pour éviter des mouvements de rotation incontrôlés du miroir lors des déplacements du dispositif en bouche, les deux articulations 5a et 5b sont munies de moyens de frein qui agissent sur les branches de l'étrier 5.

Ces moyens de frein sont, dans le mode de réalisation décrit, constitués par des bossages 8 situés d'une part sur le cadre du miroir et d'autre part sur les faces latérales du manche, de façon symétrique en regard des points de fixation des branches de l'étrier.

Ces bossages 8 sont montrés à la Figure 4 qui représente plus particulièrement la liaison entre la branche 7 de l'étrier et l'extrémité 2a du manche 2. Ainsi qu'on peut le comprendre, la rotation de la branche 7 autour du téton 7b sera ainsi freinée par la présence des bossages disposés de manière circonférentielle autour du logement du téton 7b ouvert sur la face latérale correspondante du manche.

Il convient de préciser que les branches de l'étrier présentent une certaine flexibilité et que les bossages sont réalisés de manière à ne pas constituer des saillies importantes sur les bords du miroir ou sur les faces latérales du manche, de telle sorte que le déplacement des branches de l'étrier autour de l'articulation 5b ou la rotation du miroir autour de l'articulation 5a puissent être opérés en appliquant une force légère sur lesdites branches ou ser ledit miroir.

Afin d'utiliser toutes les possibilités de prises de vues qu'offrent notamment les caméras vidéo ou afin de procéder à des corrections de visée, le dispositif de l'invention, comme on peut l'observer aux Figures 1 et 2, comprend une gorge longitudinale 20 ouverte sur le manche 2 à proximité de l'extrémité 2b, et dans laquelle est logé de manière coulissante le pied 30 de la caméra 3. De cette façon, en déplaçant la caméra le long de ladit gorge, le praticien peut, sans mouvoir le manche, obtenir des grossissements de vues ou des images panoramiques.

Par ailleurs, afin d'effectuer des corrections de l'horizontalité des images fournies par le miroir, la caméra est avantageusement montée de manière rotative autour de son axe de visée.

On peut ainsi constater que le dispositif de l'invention offre de très grandes facultés d'utilisation en autorisant notamment une infinité de réglages combinés du miroir et de la caméra.

Bien évidement, les éléments tels que miroir, manche, pied de caméra et caméra pourront être désolidarisés les uns des autres pour être soumis à stérilisation ou nettoyage.

## Revendications

1. Dispositif destiné à la prise de vues ou à la transmission d'images de corps masqués en totalité ou en partie, notamment des dents à l'intérieur de la cavité buccale, ledit dispositif comprenant un miroir (1) disposé à l'extrémité (2a) d'un manche (2), une source de lumière focalisée principalement vers ledit miroir et une caméra (3) montée sur ledit manche avec son axe de visée sensiblement dirigé vers ledite extrémité (2a), *caractérisé en ce que* ladite extrémité (2a) du manche 92) comporte une première articulation (5a) permettant de positionner ledit miroir (1) dans différents plans compris dans une plage angulaire allant de 200 à 90° par rapport au plan (I) contenant ledit manche.

2. Dispositif selon la Revendication 1, *caractérisé en ce que* le susdit axe de visée est situé dans un second plan (II) parallèle au susdit plan (1) contenant le susdit manche.

3. Dispositif selon les Revendications 1 et 2, *caractérisé en ce que* le susdit axe de visée passe par le centre du susdit miroir (1) au niveau de la susdite articulation (5a).

4. Dispositif selon l'une quelconque des Revendications 1 à 3, *caractérisé en ce que* la susdite première articulation (5a) est constituée par un étrier (5) dont les branches (6, 7) sont fixées d'une part au niveau de l'axe de rotation du susdit miroir (1) et d'autre part au susdit manche (2).

5. Dispositif selon l'une quelconque des Revendications 1 à 4 *caractérisé en ce que* ladite extrémité (2a) du manche (2) comporte en outre une seconde articulation (5b) au niveau de la fixation des susdites branches (6, 7) de l'étrier (5) au susdit manche, permettant d'amener le susdit miroir (1) hors du susdit axe de visée.

6. Dispositif selon les Revendications, 1, 4 et 5, *caractérisé en ce que* les susdites première (5a) et deuxième (5b) articulations comportent des moyens de frein (8).

7. Dispositif selon la Revendication 6, *caractérisé en ce que* lesdits moyens de frein sont constitués par des bossages (8) situés sur le susdit miroir (1) et le susdit manche (2) de façon symétrique en regard des points de fixation (6a, 7a, 6b, 7b) du susdit étrier (5).

8. Dispositif selon l'une quelconque des Revendications 1 à 3, *caractérisé en ce que* la susdite caméra (3) est montée de manière coulissante sur le susdit manche (2).

9. Dispositif selon l'une quelconque des Revendicaitons 1 à 3 et 8, *caractérisé en ce que* la susdite caméra (3) est montée de manière rotative autour de son axe de visée.

fig.1

fig.2

EP 0 420 715 A1

fig. 3

fig. 4

**Office européen des brevets**

**RAPPORT DE RECHERCHE EUROPEENNE**

Numéro de la demande

**EP 90 40 2523**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-7 233 36  (M. FAGEOT)<br>* figure 4 *<br>– – – | 1-3 | A 61 B 1/24 |
| A | FR-A-1 055 168  (E.-L. REDON et al.)<br>* page 1, colonne de gauche, ligne 39 - colonne de droite, ligne 20; figure 1 *<br>– – – | 1,2,4,5 | |
| A | DE-C-5 133 77  (C.T.N. JOHANSEN)<br>* page 1, lignes 31-65; figure 1 *<br>– – – | 1,2,6 | |
| A | EP-A-0 280 823  (FUJI OPTICAL SYSTEMS, INC)<br>* résumé; figures 1-4 *<br>– – – – – | 1,8,9 | |
| | | | **DOMAINES TECHNIQUES RECHERCHES (Int. Cl.5)**<br><br>A 61 B 1/00 |

Le présent rapport de recherche a été établi pour toutes les revendications

| Lieu de la recherche | Date d'achèvement de la recherche | Examinateur |
|---|---|---|
| Berlin | 17 décembre 90 | WEIHS J.A. |